# EUROPEAN PATENT SPECIFICATION

(11) **EP 0 575 464 B1**
(45) Date of publication and mention of the grant of the patent: **11.08.2004**
(21) Application number: 92907393.0
(22) Date of filing: 28.02.1992
(51) Int. Cl.: C12N 15/57, A61K 38/48, C12N 9/64, C12N 5/10

(54) **MODIFIED FACTOR VII**
MODIFIZIERTER FAKTOR-VII
FACTEUR VII MODIFIE

(30) Priority: 28.02.1991 US 662920
(43) Date of publication of application: 29.12.1993
(73) Proprietor: ZYMOGENETICS, INC., Seattle, WA 98105 (US); NOVO NORDISK A/S, 2880 Bagsvaerd (DK)
(72) Inventor: BERKNER, Kathleen, L., Seattle, WA 98199 (US); PETERSEN, Lars, Christian, DK-2970 Hoersholm (DK)
(74) Representative: Woods, Geoffrey Corlett
(86) International application number: PCT/US1992/001636
(87) International publication number: WO 1992/015686

(56) References cited:
- WO-A-91/11514
- The Journal of Biological Chemistry, vol. 264, no. 13, 5 May 1989, The American Society for Biochemistry and Molecular Biology, Inc., (US), E.B. WILLIAMS et al.: "Zymogen/enzyme discrimination using peptide chloromethyl ketones", pages 7536-7545, see the whole article (cited in the application)
- Proceedings of the National Academy of Sciences of the USA, vol. 84, no. 15, August 1987, (Washington, DC, US), P.J. O'HARA et al.: "Nucleotide sequence of the gene coding for human factor VII, a vitamin in K-dependent protein participating in blood coagulation", pages 5158-5162, see the whole article

## Description

### Field of the Invention

The present invention relates to proteins useful an anticoagulants. More specifically, the present invention relates to modified forms of Factor VII that inhibit blood coagulation.

### Background Of The Invention

Blood coagulation is a process consisting of a complex interaction of various blood components, or factors, which eventually gives rise to a fibrin clot. Generally, the blood components which participate in what has been referred to as the coagulation "cascade" are proenzymes or zymogens, enzymatically inactive proteins which are converted to proteolytic enzymes by the action of an activator, itself an activated clotting factor. Coagulation factors that have undergone such a conversion and generally referred to as "active factors," and are designated by the addition of a lower case "a" suffix (e.g., Factor VIIa).

Activated Factor X ("Xa") is required to convert prothrombin to thrombin, which then converts fibrinogen to fibrin as a final stage in forming a fibrin clot. There are two systems, or pathways, that promote the activation of Factor X. The "intrinsic pathway" refers to those reactions that lead to thrombin formation through utilization of factors present only in plasma. A series of protease-mediated activations ultimately generates Factor IXa which, in conjunction with Factor VIIIa, cleaves Factor X into Xa. An identical proteolysis is effected by Factor VIIa and its co-factor, tissue factor, in the "extrinsic pathway" of blood coagulation. Tissue factor is a membrane bound protein and does not normally circulate in plasma. Upon vessel disruption, however, it can complex with Factor VIIa to catalyze Factor X activation or Factor IX activation in the presence of Ca⁺⁺ and phospholipid (Nemerson and Gentry, Biochem. 25:4020-4033 (1986)). While the relative importance of the two coagulation pathways in hemostasis is unclear, in recent years Factor VII and tissue factor have been found to play a pivotal role in the regulation of blood coagulation.

Factor VII is a trace plasma glycoprotein that circulates in blood as a single-chain zymogen. The zymogen is catalytically inactive (Williams et al., J. Biol. Chem. 264:7536-7543 (1989); Rao et al., Proc. Natl. Acad. Sci. USA. 85:6687-6691 (1988)). Single-chain Factor VII may be converted to two-chain Factor VIIa by Factor Xa, Factor XIIa, Factor IXa or thrombin in vitro. Factor Xa is believed to be the major physiological activator of Factor VII. Like several other plasma proteins involved in hemostasis, Factor VII is dependent on vitamin K for its activity, which is required for the γ-carboxylation of multiple glutamic acid residues that are clustered in the amino terminus of the protein. These γ-carboxylated glutamic acids are required for the metal-associated interaction of Factor VII with phospholipids.

The conversion of zymogen Factor VII into the activated two-chain molecule occurs by cleavage of an internal peptide bond located approximately in the middle of the molecule. In human Factor VII, the activation cleavage site is at Arg₁₅₂-Ile₁₅₃ (Hagen et al., Proc. Natl. Acad. Sci. USA 83: 2412-2416 (1986); Thim et al., Biochem. 27:7785-7793 (1988). Bovine factor VII is activated by cleavage at the analogous Arg₁₅₂-Ile₁₅₃ bond (Takeya et al., J. Biol. Chem. 263: 14868-14877, 1988). In the presence of tissue factor, phospholipids and calcium ions, the two-chain Factor VIIa rapidly activates Factor X or Factor IX by limited proteolysis.

It is often necessary to selectively block the coagulation cascade in a patient. Anticoagulants such as heparin, coumarin, derivatives of coumarin, indandione derivatives, or other agents may be used, for example, during kidney dialysis, or to treat deep vein thrombosis, disseminated intravascular coagulation (DIC), and a host of other medical disorders. For example, heparin treatment or extracorporeal treatment with citrate ion (U.S. Patent 4,500,309) may be used in dialysis to prevent coagulation during the course of treatment. Heparin is also used in preventing deep vein thrombosis in patients undergoing surgery.

Treatment with heparin and other anticoagulants may, however, have undesirable side effects. Available anticoagulants generally act throughout the body, rather than acting specifically at a clot site. Heparin, for example, may cause heavy bleeding. Furthermore, with a half-life of approximately 80 minutes, heparin is rapidly cleared from the blood, necessitating frequent administration. Because heparin acts as a cofactor for antithrombin III (AT III), and AT III is rapidly depleted in DIC treatment, it is often difficult to maintain the proper heparin dosage, necessitating continuous monitoring of AT III and heparin levels. Heparin is also ineffective if AT III depletion is extreme. Further, prolonged use of heparin may also increase platelet aggregation and reduce platelet count, and has been implicated in the development of osteoporosis. Indandione derivatives may also have toxic side effects.

In addition to the anticoagulants briefly described above, several naturally occurring proteins have been found to have anticoagulant activity. For example, Reutelingsperger (U.S. Patent No. 4,736,018) isolated anticoagulant proteins from bovine aorta and human umbilical vein arteries. Maki et al. (U.S.Patent No. 4,732,891) disclose human placenta-derived anticoagulant proteins. In addition, AT III has been proposed as a therapeutic anticoagulant (Schipper et al., Lancet 1 (8069): 854-856 (1978); Jordan, U.S. Patent No. 4,386,025; Bock et al., U.S. Patent No. 4,517,294).

There is still a need in the art for improved compositions having anticoagulant activity which can be administered at relatively low doses and do not produce the undesirable side effects associated with traditional anticoagulant compositions. The present invention fulfills this need by providing anticoagulants that act specifically at sites of injury, and further provides other related advantages.

### Summary of the Invention

The present invention provides pharmaceutical compositions for use in inhibiting blood coagulation *in vivo,* the composition comprising Factor VII having at least one modification in its catalytic center, which modification substantially inhibits the ability of said modified Factor VII to activate plasma Factors X or IX, in combination with a physiologically acceptable carrier.

The invention further provides said modified Factor VII for use in therapy.

The invention further provides modified Factor VII which comprises at least one amino acid substitution, insertion or deletion in the catalytic triad of Ser, Asp and His of human Factor VII which substantially inhibits the ability of Factor VIIa to activate plasma Factor X or IX.

The invention further provides a pharmaceutical composition comprising Factor VII having at least one modification in its catalytic center, which modification substantially inhibits the ability of said modified Factor VII to activate plasma Factors X or IX, in combination with a physiologically acceptable carrier.

The Factor VII sequence has at least one amino acid modification, where the modification is selected so as to substantially reduce the ability of activated Factor VII to catalyze the activation of plasma Factors X or IX, and thus is capable of inhibiting clotting activity. The Factor VII in its modified form is capable of binding tissue factor. The modified Factor VII compositions are typically in substantially pure form.

The compositions of the invention are particularly useful in methods for treating patients when formulated into pharmaceutical compositions, where they may be given to individuals suffering from a variety of disease states to treat coagulation-related conditions. Such modified Factor VII molecules, capable of binding tissue factor but having a substantially reduced ability to catalyze activation of other factors in the clotting cascade, may possess a longer plasma half-life and thus a correspondingly longer period of anticoagulative activity when compared to anticoagulants. Among the medical indications for the subject compositions are those commonly treated with anticoagulants, such as, for example, deep vein thrombosis, pulmonary embolism, stroke, disseminated intravascular coagulation (DIC) and myocardial infarction. Thus, a method of inhibiting coagulation in a patient comprises administering a composition comprising Factor VII having at least one amino acid substitution in a catalytic triad of Ser₃₄₄, ASP₂₄₂ and His₁₉₃, in an amount sufficient to effectively inhibit coagulation.

Typically, for administration to humans the pharmaceutical compositions will comprise modified human Factor VII protein and pharmaceutically-acceptable carriers and buffers.

In preferred embodiments of human and bovine Factor VII, the active site residue Ser₃₄₄ is modified, replaced with Gly, Met, Thr, or more preferably, Ala. Such substitution could be made separately or in combination with substitution(s) at other sites in the catalytic triad, which includes His₁₉₃ and Asp₂₄₂.

In another aspect the invention relates to a polynucleotide molecule comprising two operatively linked sequence coding regions encoding, respectively, a pre-pro peptide and a gla domain of a vitamin K-dependent plasma protein, and a gla domain-less Factor VII protein, wherein upon expression said polynucleotide encodes a modified Factor VII molecule which does not significantly activate plasma Factors X or IX, and is capable of binding tissue factor. The modified Factor VII molecule expressed by this polynucleotide is a biologically active anticoagulant, that is, it is capable of inhibiting the coagulation cascade and thus the formation of a fibrin deposit or clot. To express the modified Factor VII the polynucleotide molecule is transfected into mammalian cell lines, such as, for example, BHK, BHK 570 or 293 cell lines.

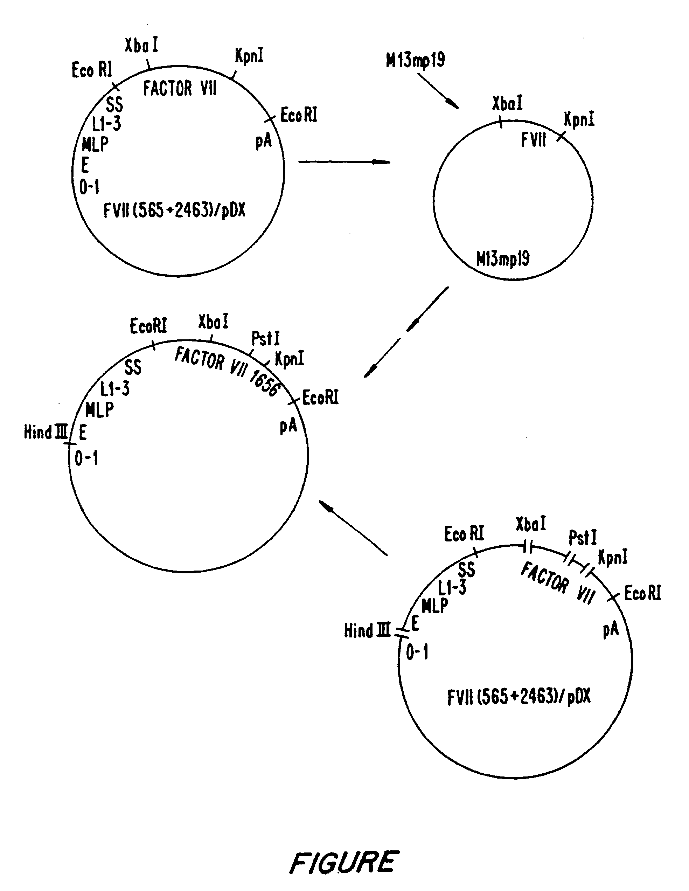

### Brief Description Of The Figure

The Figure illustrates the construction of an expression vector for a Ser₃₄₄→Ala modified Factor VII DNA sequence. Symbols used include 0-1, the 0-1 map unit sequence from adenovirus 5; E, the SV40 enhancer; MLP, the adenovirus 2 major late promotor; SS, a set of splice sites; and pA, the polyadenylation signal from SV40 in the late orientation.

### Description Of The Specific Embodiments

Novel modified Factor VII having anticoagulant activity is provided by the present invention. The modified Factor VII can be in the form of the zymogen (i.e., a single-chain molecule) or can be cleaved at its activation site. Compositions of the modified Factor VII are suitable for administration to a variety of mammals, particularly humans, to inhibit the coagulation cascade. Modified Factor VII may be administered to a patient in conjunction with or in place of other anticoagulant compounds.

Factor VII plays an important role in the coagulation cascade, particularly that involving the extrinsic pathway. Present in the circulating plasma as an inactive single chain zymogen protein, once activated, Factor VIIa, in combination with tissue factor and calcium ions, activates Factor X to Xa and activates Factor IX to IXa, with the eventual formation of a fibrin clot.

The present invention provides the ability to inhibit this sequence of events in the coagulation cascade by preventing or otherwise inhibiting the activation of Factors X and IX by Factor VIIa. Factor VII proteins of the invention have a catalytic site which is modified to decrease the catalytic activity of Factor VIIa, while the molecule retains the ability to bind to tissue factor. The modified Factor VII molecules compete with native Factor VII and/or VIIa for binding to tissue factor. AS a result, the activation of Factors X and IX is inhibited.

In one aspect of the present invention the catalytic activity of Factor VIIa is Inhibited by chemical derivatization of the catalytic center, or triad. Derivatization may be accomplished by reacting Factor VII with an irreversible inhibitor such as an organophosphor compound, a sulfonyl fluoride, a peptide halomethyl ketone or an azapeptide, or by acylation, for example. Preferred peptide halomethyl ketones include PPACK (D-Phe-Pro-Arg chloromethyl katone; see U.S. Patent No. 4,318,904, incorporated herein by reference), and DEGRcK (dansyl-Glu-Gly-Arg chloromethyl ketone).

In another aspect, the catalytic activity of Factor VIIa can also be inhibited by substituting, inserting or deleting amino acids. In preferred embodiments amino acid substitutions are made in the amino acid sequence of the Factor VII catalytic triad, defined herein as the regions which contain the amino acids which contribute to the Factor VIIa catalytic site. The substitutions, insertions or deletions in the catalytic triad are generally at or adjacent to the'amino acids which form the catalytic site. In the human and bovine Factor VII proteins, the amino acids which form.a catalytic "triad" are Ser₃₄₄, Asp₂₄₂, and His₁₉₃ (subscript numbering indicating position in the sequence). The catalytic sites in Factor VII from other mammalian species may be determined using presently available techniques including, among others, protein isolation and amino acid sequence analysis. Catalytic sites may also be determined by aligning a sequence with the sequence of other serine proteases, particularly chymotrypsin, whose active site has been previously determined (Sigler et al., J. Mol. Biol., 35:143-164 (1968), and therefrom determining from said alignment the analogous active site residues.

The amino acid substitutions, insertions or deletions are made so as to prevent or otherwise inhibit activation by the Factor VIIa of Factors X and/or IX. The Factor VII so modified should, however, also retain the ability to compete with authentic Factor VII and/or Factor VIIa for binding to tissue factor in the coagulation cascade. Such competition may readily be determined by means of, e.g., a clotting assay as described herein, or a competition binding assay using, e.g., a cell line having cell-surface tissue factor, such as the human bladder carcinoma cell line J82 (Sakai et al. J. Biol. Chem. 264: 9980-9988 (1989).

The amino acids which form the catalytic site in Factor VII, such as Ser₃₄₄, Asp₂₄₂, and His₁₉₃ in human and bovine Factor VII, may either be substituted or deleted. Within the present invention, it is preferred to change only a single amino acid, thus minimizing the likelihood of increasing the antigenicity of the molecule or inhibiting its ability to bind tissue factor, however two or more amino acid changes (substitutions, additions or deletions) may be made and combinations of substitution(s), addition(s) and deletion(s) may also be made. In a preferred embodiment for human and bovine Factor VII, Ser₃₄₄ is preferably substituted with Ala, but Gly, Met, Thr or other amino acids can be substituted. It is preferred to replace Asp with Glu and to replace His with Lys or Arg. In general, substitutions are chosen to disrupt the tertiary protein structure as little as possible. The model of Dayhoff et al. (in Atlas of Protein Structure 1978, Nat'l Biomed. Res. Found., Washington, D.C.), may be used as a guide in selecting other amino acid substitutions. One may introduce residue alterations as described above in the catalytic site of appropriate Factor VII sequence of human, bovine or other species and test the resulting protein for a desired level of inhibition of catalytic activity and resulting anticoagulant activity as described herein. For the modified Factor VII the catalytic activity will be substantially inhibited, generally less than about 5% of the catalytic activity of wild-type Factor VII of the corresponding species, more preferably less than about 1%.

The proteins of the present invention may be produced through the use of recombinant DNA techniques. In general, a cloned wild-type Factor VII DNA sequence is modified to encode the desired protein. This modified sequence is then inserted into an expression vector, which is in turn transformed or transfected into host cells. Higher eukaryotic cells, in particular cultured mammalian cells, are preferred as host cells. The complete nucleotide and amino acid sequences for human Factor VII are known. See U.S. Pat. No. 4,784,950, where the cloning and expression of recombinant human Factor VII is described. The bovine Factor VII sequence is described in Takeya et al., J. Biol. Chem. 263:14868-14872 (1988).

The amino acid sequence alterations may be accomplished by a variety of techniques. Modification of the DNA sequence may be by site-specific mutagenesis. Techniques for site-specific mutagenesis are well known in the art and are described by, for example, Zoller and Smith (DNA 3:479-488, 1984). Thus, using the nucleotide and amino acid sequences of Factor VII, one may introduce the alteration(s) of choice.

The Factor VII modified according to the present invention includes those proteins that have the amino-terminal portion (gla domain) substituted with a gla domain of one of the vitamin K-dependent plasma proteins Factor IX, Factor X, prothrombin, protein C, protein S or protein Z. The gla domains of the vitamin K-dependent plasma proteins are characterized by the presence of gamma-carboxy glutamic acid residues and are generally from about 30 to about 40 amino acids in length with C-termini corresponding to the positions of exon-intron boundaries in the respective genes. Methods for producing Factor VII with a heterologous gla domain are disclosed in U.S. Patent.No. 4,784,950.

DNA sequences for use within the present invention will typically encode a pre-pro peptide at the amino-terminus of the Factor VII protein to obtain proper post-translational processing (e.g. gamma-carboxylation of glutamic acid residues) and secretion from the host cell. The pre-pro peptide may be that of Factor VII or another vitamin K-dependent plasma protein, such as Factor IX, Factor X, prothrombin, protein C or protein S. As will be appreciated by those skilled in the art, additional modifications can be made in the amino acid sequence of the modified Factor VII where those modifications do not significantly impair the ability of the protein to act as an anticoagulant. For example, the Factor VII modified in the catalytic triad can also be modified in the activation cleavage site to inhibit the conversion of zymogen Factor VII into its activated two-chain form, as generally described in co-pending U.S.S.N. 07/471,313 and corresponding PCT publication WO-A-91 11514.

Expression vectors for use in carrying out the present invention will comprise a promoter capable of directing the transcription of a cloned gene or cDNA. Preferred promoters for use in cultured mammalian cells include viral promoters and cellular promoters. Viral promoters include the SV40 promoter (Subramani et al., Mol. Cell. Biol. 1:854-864, 1981) and the CMV promoter (Boshart et al., Cell 41:521-530, 1985). A particularly preferred viral promoter is the major late promoter from adenovirus 2 (Kaufman and Sharp, Mol. Cell. Biol. 2:1304-1319, 1982). Cellular promoters include the mouse kappa gene promoter (Bergman et al., Proc. Natl. Acad. Sci. USA 81:7041-7045, 1983) and the mouse V_{H} promoter (Loh et al., Cell 33:85-93, 1983). A particularly preferred cellular promoter is the mouse metallothionein-I promoter (Palmiter et al., Science 222:809-814, 1983). Expression vectors may also contain a set of RNA splice sites located downstream from the promoter and upstream from the insertion site for the Factor VII sequence itself. Preferred RNA splice sites may be obtained from adenovirus and/or immunoglobulin genes. Also contained in the expression vectors is a polyadenylation signal located downstream of the insertion site. Particularly preferred polyadenylation signals include the early or late polyadenylation signal from SV40 (Kaufman and Sharp, ibid.), the polyadenylation signal from the adenovirus 5 Elb region, the human growth hormone gene terminator (DeNoto et al. Nuc. Acids Res. 9:3719-3730, 1981) or the polyadenylation signal from the human Factor VII gene or the bovine Factor VII gene. The expression vectors may also include a noncoding viral leader sequence, such as the adenovirus 2 tripartite leader, located between the promoter and the RNA splice sites; and enhancer sequences, such as the SV40 enhancer.

Cloned DNA sequences are introduced into cultured mammalian cells by, for example, calcium phosphate-mediated transfection (Wigler et al., Cell 14:725-732, 1978; Corsaro and Pearson, Somatic Cell Genetics 7:603-616, 1981; Graham and Van der Eb, Virology 52d:456-467, 1973) or electroporation (Neumann et al., EMBO J. 1:841-845, 1982). To identify and select cells that express the exogenous DNA, a gene that confers a selectable phenotype (a selectable marker) is generally introduced into cells along with the gene or cDNA of interest. Preferred selectable markers include genes that confer resistance to drugs such as neomycin, hygromycin, and methotrexate. The selectable marker may be an amplifiable selectable marker. A preferred amplifiable selectable marker is a dihydrofolate reductase (DHFR) sequence. Selectable markers are reviewed by Thilly (Mammalian Cell Technology, Butterworth Publishers, Stoneham, MA,). The choice of selectable markers is well within the level of ordinary skill in the art.

Selectable markers may be introduced into the cell on a separate plasmid at the same time as the gene of interest, or they may be introduced on the same plasmid. If on the same plasmid, the selectable marker and the gene of interest may be under the control of different promoters or the same promoter, the latter arrangement producing a dicistronic message. Constructs of this type are known in the art (for example, Levinson and Simonsen, U.S. Patent 4,713,339). It may also be advantageous to add additional DNA, known as "carrier DNA," to the mixture that is introduced into the cells.

After the cells have taken up the DNA, they are grown in an appropriate growth medium, typically 1-2 days, to begin expressing the gene of interest. As used herein the term "appropriate growth medium" means a medium containing nutrients and other components required for the growth of cells and the expression of the modified Factor VII gene. Media generally include a carbon source, a nitrogen source, essential amino acids, essential sugars, vitamins, salts, phospholipids, protein and growth factors. For production of gamma-carboxylated modified Factor VII, the medium will contain vitamin K, preferably at a concentration of about 0.1 µg/ml to about 5 µg/ml. Drug selection is then applied to select for the growth of cells that are expressing the selectable marker in a stable fashion. For cells that have been transfected with an amplifiable selectable marker the drug concentration may be increased to select for an increased copy number of the cloned sequences, thereby increasing expression levels. Clones of stably transfected cells are then screened for expression of modified Factor VII.

Preferred mammalian cell lines for use in the present invention include the COS-1 (ATCC CRL 1650), baby hamster kidney (BHK) and 293 (ATCC CRL 1573; Graham et . al., J. Gen. Virol. 36:59-72, 1977) cell lines. A preferred BHK cell line is the tk⁻ ts13 BHK cell line (Waechter and Baserga, Proc. Natl. Acad. Sci. USA 79:1106-1110, 1982,), hereinafter referred to as BHK 570 cells. The BHK 570 cell line has been deposited with the American Type Culture Collection, 12301 Parklawn Dr., Rockville, MD 20852, under ATCC accession number CRL 10314. A tk⁻ ts13 BHK cell line is also available from the ATCC under accession number CRL 1632. In addition, a number of other cell lines may be used within the present invention, including Rat Hep I (Rat hepatoma; ATCC CRL 1600), Rat Hep II (Rat hepatoma; ATCC CRL 1548), TCMK (ATCC CCL 139), Human lung (ATCC HB 8065), NCTC 1469 (ATCC CCL 9.1), CHO (ATCC CCL 61) and DUKX cells (Urlaub and Chasin, Proc. Natl. Acad. Sci. USA 77:4216-4220, 1980).

Modified Factor VII produced according to the present invention may be purified by affinity chromatography on an anti-Factor VII antibody column. The use of calcium-dependent monoclonal antibodies, as described by Wakabayashi et al., J. Biol. Chem. 261:11097-11108, (1986) and Thim et al., Biochem. 27: 7785-7793, (1988), is particularly preferred. Additional purification may be achieved by conventional chemical purification means, such as high performance liquid chromatography. Other methods of purification, including barium citrate precipitation, are known in the art, and may be applied to the purification of the novel modified Factor VII described herein (see, generally, Scopes, R., Protein Purification, Springer-Verlag, N.Y., 1982). Substantially pure modified Factor VII of at least about 90 to 95% homogeneity is preferred, and 98 to 99% or more homogeneity most preferred, for pharmaceutical uses. Once purified, partially or to homogeneity as desired, the modified Factor VII may then be used therapeutically.

Within one embodiment of the invention the modified Factor VII is cleaved at its activation site to convert it to its two-chain form. Activation may be carried out according to procedures known in the art, such as those disclosed by Osterud, et al., Biochemistry 11:2853-2857 (1972); Thomas, U.S. Patent No. 4,456,591; Hedner and Kisiel, J. Clin. Invest. 71:1836-1841 (1983); or Kisiel and Fujikawa, Behring Inst. Mitt. 73:29-42 (1983). The resulting modified "Factor VIIa" is then formulated and administered as described below.

The modified Factor VII or VIIa molecules of the present invention and pharmaceutical compositions thereof are particularly useful for administration to humans to treat a variety of conditions involving intravascular coagulation. For instance, although deep vein thrombosis and pulmonary embolism can be treated with conventional anticoagulants, the modified Factor VII described herein may be used to prevent the occurrence of thromboembolic complications in identified high risk patients, such as those undergoing surgery or those with congestive heart failure. Since modified Factor VII is more selective than heparin, generally binding only tissue factor which has been exposed at sites of injury, and because modified Factor VII does not destroy other coagulation proteins, it will be more effective and less likely to cause bleeding complications than heparin when used prophylactically for the prevention of deep vein thrombosis. The dose of modified Factor VII for prevention of deep vein thrombosis is in the range of about 50 µg to 25 mg/day, preferably 1 to 10 mg/day for a 70 kg patient, and administration should begin at least about 6 hours prior to surgery and continue at least until the patient becomes ambulatory. In established deep vein thrombosis and/or pulmonary embolism, the dose of modified Factor VII ranges from about 50 µg to 25 mg as a loading dose followed by maintenance doses ranging from about 500 µg to 10 mg/day, depending on the weight of the patient and the severity of the condition. Because of the lower likelihood of bleeding complications from modified Factor VII infusions, modified Factor VII can replace or lower the dose of heparin during or after surgery in conjunction with thrombectomies or embolectomies.

The modified Factor VII compositions of the present invention will also have substantial utility in the prevention of cardiogenic emboli and in the treatment of thrombotic strokes. Because of its low potential for causing bleeding complications and its selectivity, modified Factor VII can be given to stroke victims and may prevent the extension of the occluding arterial thrombus. The amount of modified Factor VII administered will vary with each patient depending on the nature and severity of the stroke, but doses will generally be in the range of those suggested below.

Pharmaceutical compositions of modified Factor VII provided herein will be a useful treatment in acute myocardial infarction because of the ability of modified Factor VII to inhibit in vivo coagulation. Modified Factor VII can be given with tissue plasminogen activator or streptokinase during the acute phases of the myocardial infarction. In acute myocardial infarction, the patient is given a loading dose of at least about 1 to 25 mg of modified Factor VII, followed by maintenance doses of about 500 µg to about 10 mg/day.

The modified Factor VII of the present invention is useful in the treatment of disseminated intravascular coagulation (DIC). Patients with DIC characteristically have widespread microcirculatory thrombi and often severe bleeding problems which result from consumption of essential clotting factors. Because of its selectivity, modified Factor VII will not aggravate the bleeding problems associated with DIC, as do conventional anticoagulants, but will retard or inhibit the formation of additional microvascular fibrin deposits.

The pharmaceutical compositions are intended for parenteral, topical or local administration for prophylactic and/or therapeutic treatment. Preferably, the pharmaceutical compositions are administered parenterally, i.e., intravenously, subcutaneously, or intramuscularly. Thus, this invention provides compositions for parenteral administration which comprise a solution of the modified Factor VII molecules dissolved in an acceptable carrier, preferably an aqueous carrier. A variety of aqueous carriers may be used, e.g., water, buffered water, 0.4% saline, 0.3% glycine and the like. The modified Factor VII molecules can also be formulated into liposome preparations for delivery or targeting to sites of injury. Liposome preparations are generally described in, e.g., U.S. 4,837,028, U.S. 4,501,728, and U.S. 4,975,282. The compositions may be sterilized by conventional, well known sterilization techniques. The resulting aqueous solutions may be packaged for use or filtered under aseptic conditions and lyophilized, the lyophilized preparation being combined with a sterile aqueous solution prior to administration. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride, etc. The concentration of modified Factor VII in these formulations can vary widely, i.e., from less than about 0.5%, usually at or at least about 1% to as much as 15 or 20% by weight and will be selected primarily by fluid volumes, viscosities, etc., in accordance with the particular mode of administration selected.

Thus, a typical pharmaceutical composition for intravenous infusion could be made up to contain 250 ml of sterile Ringer's solution, and 10 mg of modified Factor VII. Actual methods for preparing parenterally administrable compounds will be known or apparent to those skilled in the art and are described in more detail in for example, Remington's Pharmaceutical Science, 16th ed., Mack Publishing Company, Easton, PA (1982).

The compositions containing the modified Factor VII molecules can be administered for prophylactic and/or therapeutic treatments. In therapeutic applications, compositions are administered to a patient already suffering from a disease, as described above, in an amount sufficient to cure or at least partially arrest the disease and its complications. An amount adequate to accomplish this is defined as "therapeutically effective dose." Amounts effective for this use will depend on the severity of the disease or injury and the weight and general state of the patient, but generally range from about 0.05 mg to about 25 mg of modified Factor VII per day for a 70 kg patient, with dosages of from about 0.5 mg to about 10 mg of modified Factor VII per day being more commonly used. It must be kept in mind that the materials of the present invention may generally be employed in serious disease or injury states, that is, life-threatening or potentially life threatening situations. In such cases, in view of the minimization of extraneous substances and general lack of immunogenicity of modified human Factor VII in humans, it is possible and may be felt desirable by the treating physician to administer substantial excesses of these modified Factor VII compositions.

In prophylactic applications, compositions containing the modified Factor VII are administered to a patient susceptible to or otherwise at risk of a disease state or injury to enhance the patient's own anticoagulative capabilities. Such an amount is defined to be a "prophylactically effective dose." In this use, the precise amounts again depend on the patient's state of health and weight, but generally range from about 0.1 mg to about 25 mg per 70 kilogram patient, more commonly from about 0.5 mg to about 10 mg per 70 kg of body weight.

Single or multiple administrations of the compositions can be carried out with dose levels and pattern being selected by the treating physician. For ambulatory patients requiring daily maintenance levels, the modified Factor VII may be administered by continuous infusion using a portable pump system, for example. In any event, the pharmaceutical formulations should provide a quantity of modified Factor VII of this invention sufficient to effectively treat the patient.

The following examples are offered by way of illustration, not by way of limitation.

### EXAMPLE I

### Expression of Ser₃₄₄→Ala₃₄₄ Factor VII

To generate the Ser₃₄₄→Ala Factor VII active site mutant, plasmid FVII(565+2463)/ pDX (U.S. Patent No. 4,784,950; deposited with the American Type Culture Collection under accession number 40205) was digested with Xba I and Kpn I, and the resulting 0.6 kb fragment, comprising the coding region for serine 344, was recovered. This fragment was cloned into Xba I, Kpn I-digested M13mp19 as shown in the Figure. This manipulation and subsequent steps described below were generally performed according to standard protocols (as described, for example, by Maniatis et al., Molecular Cloning, A Laboratory Manual, Cold Spring Harbor Laboratory Press, Cold Spring Harbor, N.Y. (1982).

Mutagenesis was carried out on the M13 template according to the methods of Zoller and Smith, supra, using the mutagenic oligonucleotide ZC1656 (5' TGG GCC TCC GGC GTC CCC CTT 3') and the "universal" second primer ZC87 (5' TCC CAG TCA CGA CGT 3'). Reaction products were screened using kinased ZC1656. Positive plaques were picked, and template DNA was prepared and sequenced from the Pst I site at 1077 to the Kpn I site at 1213. Sequence analysis confirmed the presence of the desired mutation. The mutant clone was designated 1656.

An expression vector was then constructed using the 1656 clone. The mutagenized sequence was isolated from the M13 vector as a -0.14 kb Pst I-Kpn I fragment. This fragment was ligated to the 1.7 kb Hind III-Xba I fragment from FVII(565+2463)/pDX, the 0.5 kb Xba I-Pst I fragment from FVII(565+2463)/pDX, and the 4.3 kb Kpn I-Hind III fragment from FVII(565+2463)/pDX, as shown in the Figure. The presence of the desired mutant sequence was confirmed by digesting mutant and wild-type clones with Pst I, and a mutant Factor VII insert in M13 with Kpn I and Xba I, preparing Southern blots of the digested DNA, and probing the blots with radiolabeled ZC1656.

The baby hamster kidney cell line BHK 570 (deposited with the American Type Culture Collection under accession number 10314) was transfected with two isolates (designated #544 and #545) of the 1656 expression vector. The cells were prepared by diluting a confluent 10 cm plate of BHK 570 cells 1:10 into five 10 cm plates in non-selective medium (Dulbecco's modified Eagle's medium [DMEM] containing 10% fetal bovine serum and 1% PSN antibiotic mix [GIBCO Life Technologies, Gaithersburg, MD]). After 24 hours, when the cells had reached 20-30% confluency, they were co-transfected with one isolate of the expression vector encoding the 1656 mutation, plasmid p486 (comprising the Adenovirus 5 ori, SV40 enhancer, Adenovirus 2 major late promotor, Adenovirus 2 tripartite leader, 5' and 3' splice sites, the DHFR^{r} cDNA and SV40 polyadenylation signal in pML-1 • (Lusky and Botchan, Nature 293: 79-81, (1981)) and 10 µg of carrier DNA (sonicated salmon sperm DNA) as shown in Table 1. The DNA was added to a 15 ml tube, then 0.5 ml of 2X Hepes (25 g Hepes, 40 g NaCl, 1.8 g KCl, 0.75 g Na₂HPO₄·2H₂O, 5 g dextrose diluted to 2.5 l with distilled water and pH adjusted to pH 6.95-7.0) was added and the tubes were mixed. The DNA in each tube was precipitated by the addition of 0.5 ml of 0.25 M CaCl₂ while air was bubbled through the DNA/Hepes solution with a pasteur pipet. The tubes were then vortexed, incubated at room temperature for 15 minutes, and vortexed again. The DNA mixtures were then added dropwise onto the plates of cells with a pipette. The plates were swirled and incubated at 37°C for 4-6 hours. After incubation, 2 ml of 20% glycerol diluted in Tris-saline (0.375 g KCl, 0.71 g Na₂HPO₄, 8.1 g NaCl, 3.0 g Tris-HCl, 0.5 g sucrose, diluted in a total of 1 liter and pH adjusted to pH 7.9) was then added to each plate. The plates were swirled and left at room temperature for two minutes. The medium was then removed from the plates and replaced with 2 ml of Tris-saline. The plates were left at room temperature for 2 minutes, then the Tris-saline was removed and replaced with 10 ml of non-selective medium. The plates were incubated at 37°C for two days.

**Table 1**

| Transfection* | | | | |
|---|---|---|---|---|
| | 544 | 545 | 544 Control | 545 Control |
| Plasmid Name | | | | |
| Clone 544 | 15 µl | --- | 15 µl | --- |
| Clone 545 | --- | 30 µl | --- | 30 µl |
| p486 | 1.5 µl | 1.5 µl | --- | --- |
| Carrier DNA | 1.6 µl | 1.6 µl | 1.6 µl | 1.6 µl |

| | | | | |
|---|---|---|---|---|
| * DNA concentrations used were: clone 544, 0.7 µg/µl: clone 545, 0.3 µg/µl; p486, 1.49 µg/µl. | | | | |

After the two day incubation, the cells were diluted in selection medium (DMEM containing 10% dialyzed fetal bovine serum, 1% PSN antibiotic mix and 150 nM methotrexate) and plated at dilutions of 1:100, 1:250 and 1:500 in maxi plates. The plates were incubated at 37°C for one week. After one week, the medium was changed and replaced with selection medium, and the plates were checked for colony formation.

Eight days later, after colony formation, twelve colonies were randomly chosen from the 1:500 dilution plates of the #544 and #545 transfection plates. Each clone was plated into one well of a 6-well plate and grown in selection medium. After seven days, the plates were confluent, and the clones were each split into 10 cm plates in selection medium.

The clones described above and control cells transfected to express wild-type factor VII were metabolically labeled with ³⁵S-Methionine-Cysteine Protein Labeling Mix (NEN DuPont Biotechnology Systems, Wilmington, DE). The clones were grown and prepared for a pulse label experiment in selective medium. The cells were rinsed with phosphate buffered saline (Sigma, St. Louis, MO) and pulsed for four hours in 20 µCi/ml ³⁵S-Cys-³⁵S-Met. After four hours, supernatants and cells were harvested. The cells were lysed essentially as described by Lenk and Penman (Cell 16: 289-302, (1979)) and 400 µl of each lysate and precleared with 50 µl of staph A (Sigma, St. Louis, MO).

Samples from the metabolically labeled cells were radioimmunoprecipitated (RIP) by first incubating the samples with 6 µl of anti-Factor VII polyclonal antisera for four hours. Sixty microliters of washed staphylococcal protein A was added to each sample, and the samples were rocked for 1.5 hours at 4°C. The samples were centrifuged, and the supernatant was removed. The pellets were washed twice in 0.7 M RIPA buffer (10 mM Tris, pH 7.4, 1% deoxycholic acid [Calbiochem Corp., La Jolla, CA], 1% Triton X-100, 0.1% SDS, 5 mM EDTA, 0.7 M NaCl) and once in 0.15 M RIPA buffer (10 mM Tris, pH 7.4, 1% deoxycholic acid [Calbiochem Corp., La Jolla, CA], 1% Triton X-100, 0.1 SDS, 5 mM EDTA, 0.15 M NaCl). One hundred microliters of 1X SDS dye (50 mM Tris-HCl, pH 6.8, 100 mM dithiothreitol, 2% SDS, 0.1% bromphenol blue, 10% glycerol) was added to each sample, and the samples were boiled for 5 minutes followed by centrifugation to remove the protein A. Fifty microliters of each sample was run on a 10% polyacrylamide gel. Results showed that 9 of 10 clones secreted modified Factor VII.

### EXAMPLE II

### ANTICOAGULANT ACTIVITY OF MODIFIED FACTOR VII

The ability of the modified Factor VII protein to inhibit clotting was measured in a one-stage clotting assay using wild-type Factor VII as a control. Recombinant proteins were prepared essentially as described above from cells cultured in media containing 5µg/ml vitamin K. Varying amounts of the modified Factor VII (from clone 544) or recombinant wild-type Factor VII were diluted in 50 mM Tris pH 7.5, 0.1% BSA to 100 µl. The mixtures were incubated with 100 µl of Factor VII-deficient plasma (George King Bio-Medical Inc., Overland Park, KS) and 200 µl of thromboplastin C (Dade, Miami, FL; contains rabbit brain thromboplastin and 11.8 mM Ca⁺⁺). The clotting assay was performed in an automatic coagulation timer (MLA Electra 800, Medical Laboratory Automation Inc., Pleasantville, NY), and clotting times were converted to units of Factor VII activity using a standard curve constructed with 1:5 to 1:640 dilutions of normal pooled human plasma (assumed to contain one unit per ml Factor VII activity; prepared by pooling citrated serum from healthy donors). Using this assay the preparations of modified Factor VII exhibited no detectable coagulant activity. Table 2 shows results of the assay in terms of clotting times for control (untransfected) BHK cell-conditioned media (+/- vitamin K), wild-type Factor VII and two isolates of cells expressing the modified Factor VII. Factor VII activity is seen as a reduction in clotting time over control samples.

**Table 2**

| Sample | Dilution | Clotting Time (sec.) |
|---|---|---|
| Control +K | 1:5 | 33.1 |
| | 1:10 | 33.4 |
| | | |
| Control -K | 1:5 | 34.3 |
| | 1:10 | 33.2 |
| | | |
| Wild-type Factor VII | 1:20 | 19.0 |
| | 1:40 | 21.5 |
| | 1:80 | 23.3 |
| | | |
| Modified Factor VII (#6) | 1:1 | 33.5 |
| | | |
| Modified Factor VII (#10) | 1:1 | 32.5 |

To determine the effect of the modified Factor VII on plasma factor substrates, preparations of modified Factor VII and recombinant wild-type or native Factor VII are incubated with either Factor X or Factor IX and the activation thereof monitored by clotting assays or polyacrylamide gel electrophoresis.

### EXAMPLE III

### Ability of Modified Factor VII to Bind Tissue Factor

The ability of the modified Factor VII to compete with wild-type Factor VII for tissue factor and inhibit its clotting activity was assessed in a one-step clotting assay in the presence of a limiting amount of tissue factor (thromboplastin).

Clotting times were determined in a one-step assay similar to that described in Example II. A limited amount of tissue factor, a constant amount of wild type Factor VII, and increasing amounts of variant Factor VII were used in the mixing experiments. An inhibition of Factor VII/VIIa procoagulant activity would be seen as an increase in clotting time in assays containing increasing amounts of variant Factor VII.

The amount of Factor VII activity in the test samples was calculated as a percentage of a standard curve that measured Factor VII activity in normal pooled plasma. The standard curve for Factor VII activity was generated using serial dilutions of normal pooled plasma in phosphate buffered solution (PBS) that ranged from 1:5 to 1:640. For this purpose it was assumed that normal plasma contains approximately 500 ng/ml of Factor VII and this was considered to be one unit of activity. A mixture of 100 µl Factor VII-deficient plasma, 100 µl plasma dilution and 200 µl of thromboplastin-C (Dade, Miami, FL.) was used to measure clotting time on a MLA Electra 800 automatic timer. To establish the standard curve, the results were graphed as percentage of activity (1:5 = 100% activity) versus clotting time in seconds.

The assay required that the medium containing the wild type and variant Factor VII be composed of less than one percent serum. The dilutions were made in PBS so that clotting times would fall along the standard curve. A minimum dilution of 1:2 was typical. The final volume was 100 µl. Two different human Factor VII Ser₃₄₄ → Ala variants, designated clones "#10" and "#6" were tested in the experiments. The results, set forth in the Table below, show that as the amount of Factor VII variant increased, the percent of Factor VIIa activity decreased.

**Table 3:**

| Results of mixing assay with Ser344 → Ala Variants (B4A1 (wild type) medium was used as 100% activity at 10 µl/reaction) | | | | |
|---|---|---|---|---|
| Percent Ser344 → Ala Clone No. | Variant medium amount | B4A1 medium amount | BHK Control* | FVIIa Activity |
| #10 | 10 µl | 10 µl | 0 | 70 |
| #10 | 20 µl | 10 µl | 0 | 51 |
| #10 | 30 µl | 10 µl | 0 | 43 |
| #10 | 40 µl | 10 µl | 0 | 34 |
| #10 | 50 µl | 10 µl | 0 | 28 |
| #10 (-K)^{§} | 20 µl | 10 µl | 0 | 78 |
| #6 | 10 µl | 10 µl | 0 | 74 |
| #6 | 20 µl | 10 µl | 0 | 56 |
| #6 | 30 µl | 10 µl | 0 | 46 |
| #6 | 40 µl | 10 µl | 0 | 41 |
| #6 | 50 µl | 10 µl | 0 | 32 |
| #6 (-K) | 20 µl | 10 µl | 0 | 85 |
| BHK control | 0 | 10 µl | 20 µl | 91 |
| BHK control (-K) | 0 | 10 µl | 20 µl | 107 |

| | | | | |
|---|---|---|---|---|
| * Untransfected conditioned medium | | | | |
| § For expression of the Factor VII variant, cells were grown in the presence of vitamin K, except where noted "(-K)". | | | | |

These experiments showed that variants of Factor VII having a Ser₃₄₄ → Ala substitution competed with native Factor VII in a dose dependent fashion and inhibited the procoagulant activity of native Factor VII/VIIa. It can thus be concluded that Ser₃₄₄ → Ala variant human Factor VII competes with native human Factor VIIa and consequently inhibits activation of Factor X and/or IX in human plasma.

### EXAMPLE IV

### Reaction of Factor VII with PPACK

Recombinant Factor VII was produced in transfected baby hamster kidney cells. The protein was purified and activated as disclosed by Thim et al. (Biochemistry 27: 7785-7793, 1988), Brinkous et al. d(Proc. Natl.Acad. Sci. USA 86: 1382-1386, 1989) and Bjoern and Thim (Res. Discl. No. 269, 564, 1986). The cell culture medium was recovered, filtered and diluted to reduce sale concentration. The diluted medium was then fractionated by anion exchange chromatography using an elution buffer containing CaCl₂. The Factor VII fraction was recovered and further purified by immunochromatography using a calcium-dependent anti-Factor VII monoclonal antibody. Additional purification was carried out using two anion exchange chromatography steps wherein Factor VII was eluted using CaCl₂ and NaCl, respectively. Factor VIIa was recovered in the final eluate.

Recombinant Factor VIIa (1 µM) in 50 mM Tris-HCl, 100 mM NaCl, 5 mM CaCl₂, ph 7.4 was incubated with 20 µM PPack (D-Phenylalanyl-Prolyl-Arginyl Chloromethyl Ketone; Calbiochem, La Jolla, CA) for 5, 20 and 60 minutes. Buffer containing the chromogenic substrate S2288 (H-D-Isoleucine-L-Prolyl-L-Arginine p-nitroanilide; Kabi Vitrum AB, Molndal, Sweden) was then added to obtain a 2.5 fold dilution and a final concentration of 0.3 mM S2288. The generation of p-nitroaniline was measured and compared to results using untreated Factor VIIa as a control. The results indicated that Factor VIIa is fully inactivated after about 60 minutes under these reaction conditions.

### EXAMPLE V

### Reaction of Factor VII with DEGRcK

Recombinant Factor VIIa was prepared as described in Example IV. Factor VIIa (1 µM) was incubated with 0.7 mM DEGRck (dansyl-Glu-Gly-Arg chloromethyl ketone; Calbiochem) at 37°C for one hour in a total volume of 200 µl of 0.05 M Tris-HCL, 0.1 M NaCl, 0.5% BSA, pH 7.5. Following incubation, the mixture was dialyzed overnight at 4°C versus two liters of 0.05 M Tris-HCl, 0.1 M NaCl, pH 7.5

Clotting activity of the modified Factor VII was assessed by monitoring the change in clotting time of hereditary Factor VII deficient plasma. 100 µl samples were combined with 100 µl each of Factor VII deficient plasma, human brain thromboplastin (prepared as described by Nawroth et al., Thromb. Res. 44: 625-637, 1986) and 25 mM CaCl₂. Clotting times were converted to units of Factor VII activity from a standard curve constructed with 1:5 to 1:200 dilutions of normal pooled human plasma. Incubation of Factor VIIa with DEGRck was found to result in a complete loss of Factor VII clotting activity.

It in evident from the foregoing that compositions of Factor VII having modified catalytic sites are provided which are able to bind tissue factor yet are substantially unable to activate Factors X and IX. As modified Factor VII acts specifically to interrupt the clotting cascade without degrading or consuming clotting factors, it can be expected that administration of modified Factor VII preparations will be accompanied by fewer undesirable side effects than experienced with current therapies. Further, the modified Factor VII described herein may readily be produced by recombinant means: Thus efficacy, convenience and economics of lower dosages and less frequent administration, and a relative lack of toxicity are among the advantages conferred by the compositions of the present invention.

## Claims

1. A pharmaceutical composition for use in inhibiting blood coagulation in vivo, the composition comprising Factor VII having at least one modification in its catalytic center, which modification substantially inhibits the ability of said modified Factor VII to activate plasma Factors X or IX, in combination with a physiologically acceptable carrier.

2. The pharmaceutical Composition according to claim 1, wherein said modification comprises reaction of Factor VII with a serine protease inhibitor.

3. The pharmaceutical composition according to claim 2, wherein said inhibitor is an organophosphor compound a sulfanyl fluoride, a peptide halomethyl ketone or an azapeptide.

4. The pharmaceutical composition according to claim 2, wherein said inhibitor is a peptide halomethyl ketone selected from D-Phe-Pro-Arg chloromethyl ketone or Dansyl-Glu-Gly-Arg chloromethyl ketone.

5. The pharmaceutical composition according to claim 1, wherein the Factor VII modification comprises at least one amino acid substitution, insertion or deletion in a catalytic triad of Ser, Asp and His.

6. The pharmaceutical composition according to claim 5, wherein the Factor VII is modified by a substitution at residue Ser₃₄₄.

7. The pharmaceutical composition according to any one of the preceding claims, wherein the modified factor VII is human.

8. The modified Factor VII as defined in any one of the preceding claims for use in therapy.

9. Use of the modified Factor VII as defined in any one of claims 1 to 7 in the manufacture of a medicament for inhibiting blood coagulation.

10. Modified Factor VII which comprises at least one amino acid substitution, insertion or deletion in the catalytic triad of Ser, Asp and His of human Factor VII which substantially inhibits the ability or Factor VIIa to activate plasma Factor X or IX.

11. The modified Factor VII of claim 10, wherein the catalytic triad is comprised of Ser₃₄₄, Asp₂₄₂, and His₁₉₃.

12. The modified Factor VII of claim 11, wherein the amino acid modification is a substitution.

13. The modified Factor VII of claim 12, wherein the substitution is at Ser₃₄₄.

14. The modified Factor VII of claim 13, wherein Ala, Gly, Met or Thr is substituted for Ser₃₄₄.

15. The modified Factor VII of claim 14, wherein Ala is substituted for Ser₃₄₄.

16. The modified Factor VII of claim 12, wherein Glu is substituted for Asp₂₄₂.

17. The modified Factor VII of claim 12, wherein Lys or Arg is substituted for His₁₉₃.

18. The modified Factor VII of any one of claims 10 to 17, cleaved at its activation site.

19. The modified Factor VII of any one of claims 10 to 17, which binds tissue factor.

20. The modified Factor VII of any one of claims 10 to 17, which competes with wild-type Factor VIIa for binding to tissue factor.

21. Modified Factor VII of any one of claims 10 to 20 for use in therapy.

22. Use of the modified Factor VII of any one of claims 10 to 20 in the manufacture of a pharmaceutical composition for inhibiting blood coagulation.

23. A polynucleotide molecule comprising two operatively linked sequence coding regions encoding, respectively, a pre-pro peptide and a gla domain of a vitamin K-dependent plasma protein, and a gla domain-less Factor VII having at least one amino acid modification in a catalytic triad of Ser, Asp and His, wherein upon expression said polynucleotide encodes a modified Factor VII molecule that has a substantially reduced ability to activate plasma Factors X or IX.

24. The polynucleotide molecule according to claim 23, wherein the catalytic triad having the amino acid modification is comprised of Ser₃₄₄, Asp₂₄₂, and His₁₉₃.

25. A mammalian cell line transfected with the polynucleotide molecule of claim 23 or claim 24.

26. The cell line according to claim 25, wherein the catalytic triad is Ser₃₄₄, Asp₂₄₂ and His₁₉₃ of human Factor VII.

27. A pharmaceutical composition comprising Factor VII having at least one modification in its catalytic center, which modification substantially inhibits the ability of said modified Factor VII to activate plasma Factors X or LX, in combination with a physiologically acceptable carrier.

28. The pharmaceutical composition according to claim 27, wherein said modification comprises reaction of Factor VII with a serine protease inhibitor.

29. The pharmaceutical composition according to claim 28, wherein said inhibitor is an organophosphor compound, a sulfanyl fluoride, a peptide halomethyl ketone or an azapeptide.

30. The pharmaceutical composition according to claim 28, wherein said inhibitor is a peptide halomethyl ketone selected from D-Phe-Pro-Arg chloromethyl ketone or Dansyl-Glu-Gly-Arg chloromethyl ketone.

31. The pharmaceutical composition according to claim 27, wherein the Factor VII modification comprises at least one amino acid substitution, insertion or deletion in a catalytic triad of Ser, Asp and His.

32. The pharmaceutical composition according to claim 31, wherein the Factor VII is modified by a substitution at residue Ser₃₄₄.

33. The pharmaceutical composition according to any one of claims 27 to 32, wherein the modified Factor VII is human.

## Patentansprüche

1. Pharmazeutische Zusammensetzung zur Inhibierung der Blutgerinnung in vivo, wobei die Zusammensetzung Faktor VII,
der mindestens eine Modifikation in seinem katalytischen Zentrum hat, wobei die Modifikation im Wesentlichen die Fähigkeit des modifizierten Faktors VII, die Plasmafaktoren X oder IX zu aktivieren, inhibiert, in Kombination mit einem physiologisch annehmbaren Träger umfasst.

2. Pharmazeutische Zusammensetzung nach Anspruch 1, wobei die Modifikation eine Reaktion von Faktor VII mit einem Serinproteaseinhibitor umfasst.

3. Pharmazeutische Zusammensetzung nach Anspruch 2, wobei der Inhibitor eine Organophosphorverbindung, ein Sulfanylfluorid, ein Peptidhalogenmethylketon oder ein Azapeptid ist.

4. Pharmazeutische Zusammensetzung nach Anspruch 2, wobei der Inhibitor ein Peptidhalogenmethylketon, ausgewählt aus D-Phe-Pro-Arg-chlormethylketon oder Dansyl-Glu-Gly-Arg-chlormethylketon, ist.

5. Pharmazeutische Zusammensetzung nach Anspruch 1, wobei die Faktor VII-Modifikation mindestens eine Aminosäure-Substitution, -Insertion oder -Deletion in einer katalytischen Triade aus Ser, Asp und His umfasst.

6. Pharmazeutische Zusammensetzung nach Anspruch 5, wobei der Faktor VII durch eine Substitution am Rest Ser₃₄₄ modifiziert ist.

7. Pharmazeutische Zusammensetzung nach einem der vorangehenden Ansprüche, wobei der modifizierte Faktor VII ein humaner ist.

8. Modifizierter Faktor VII nach einem der vorangehenden Anspruche zur Verwendung in einer Therapie.

9. Verwendung des modifizierten Faktors VII nach einem der Ansprüche 1 bis 7 bei der Herstellung eines Medikaments zur Inhibierung der Blutgerinnung.

10. Modifizierter Faktor VII, der mindestens eine Aminosauresubstitution, -Insertion oder -Deletion in der katalytischen Triade aus Ser, Asp und His des humanen Faktors VII umfasst, der im Wesentlichen die Fähigkeit von Faktor VIIa, Plasma-Faktor X oder IX zu aktivieren, inhibiert.

11. Modifizierter Faktor VII nach Anspruch 10, wobei die katalytische Triade aus Ser₃₄₄, Asp₂₄₄ und His₁₉₃ besteht.

12. Modifizierter Faktor VII nach Anspruch 11, wobei die Aminosauremcdifikation eine Substitution ist.

13. Modifizierter Faktor VII nach Anspruch 12, wobei die Substitution an Ser₃₄₄ ist.

14. Modifizierter Faktor VII nach Anspruch 13, wobei Ala, Gly, Met oder Thr für Ser₃₄₄ eingesetzt ist.

15. Modifizierter Faktor VII nach Anspruch 14, wobei Ala für Ser₃₄₄ eingesetzt ist.

16. Modifizierter Faktor VII nach Anspruch 12, wobei Glu für Asp₂₄₂ eingesetzt ist.

17. Modifizierter Faktor VII nach Anspruch 12, wobei Lys oder Arg für His₁₉₃ eingesetzt ist.

18. Modifizierter Faktor VII nach einem der Ansprüche 10 bis 17, der an seiner Aktivierungsstelle gespalten ist.

19. Modifizierter Faktor VII nach einem der Ansprüche 10 bis 17, welcher Gewebefaktor bindet.

20. Modifizierter Faktor VII nach einem der Ansprüche 10 bis 17, der mit Wildtyp-Faktor VIIa um Bindung an Gewebefaktor im Wettbewerb steht.

21. Modifizierter Faktor VII nach einem der Ansprüche 10 bis 20 zur Verwendung in einer Therapie.

22. Verwendung des modifizierten Faktors VII nach einem der Ansprüche 10 bis 20 in der Herstellung einer pharmazeutischen Zusammensetzung zur Inhibierung der Blutgerinnung.

23. Polynukleotidmolekül, umfassend zwei funktionell gebundene sequenzkodierende Regionen, die für ein Pre-Pro-Peptid und eine Gla-Domäne eines Vitamin K-abhängigen Plasmaproteins bzw. einen Faktor VII ohne Gla-Domäne, der mindestens eine Aminosäuremodifikation in einer katalytischen Triade aus Ser, Asp und His hat, kodieren, wobei bei Expression das Polynukleotid für ein modifiziertes Faktor VII-Molekül kodiert, das eine wesentlich reduzierte Fähigkeit, die Plasmafaktoren X oder IX zu aktivieren, hat.

24. Polynukleotidmolekül nach Anspruch 23, wobei die katalytische Triade, die die Aminosäuremodifikation hat, aus Ser₃₄₄, Asp₂₄₂ und His₁₉₃ besteht.

25. Säugetierzelllinie, die mit dem Polynukleotidmolekül nach Anspruch 23 oder Anspruch 24 transfiziert ist.

26. Zelllinie nach Anspruch 25, wobei die katalytische Triade Ser₃₄₄, Asp₂₄₂ und His₁₉₃ des humanen Faktors VII ist.

27. Pharmazeutische Zusammensetzung, die Faktor VII,
der mindestens eine Modifikation in seinem katalytischen Zentrum hat, wobei die Modifikation die Fähigkeit des modifizierten Faktors VII, die Plasmafaktoren X oder IX zu aktivieren, wesentlich inhibiert, in Kombination mit einem physiologisch annehmbaren Träger umfasst.

28. Pharmazeutische Zusammensetzung nach Anspruch 27, wobei die Modifikation eine Reaktion von Faktor VII mit einem Serinproteaseinhibitor umfasst.

29. Pharmazeutische Zusammensetzung nach Anspruch 28, wobei der Inhibitor eine Organophosphorverbindung, ein Sulfanylfluorid, ein Peptidhalogenmethylketon oder ein Azapeptid ist.

30. Pharmazeutische Zusammensetzung nach Anspruch 28, wobei der Inhibitor ein Peptidhalogenmethylketon, ausgewählt aus D-Phe-Pro-Arg-chlormethylketon und Dansyl-Glu-Gly-Arg-chlormethylketon, ist.

31. Pharmazeutische Zusammensetzung nach Anspruch 27, wobei die Faktor VII-Modifikation mindestens eine Aminosäuresubstitution, -Insertion oder -Deletion in einer katalytischen Triade aus Ser, Asp und His umfasst.

32. Pharmazeutische Zusammensetzung nach Anspruch 31, wobei der Faktor VII durch eine Substitution am Rest Ser₃₄₄ modifiziert ist.

33. Pharmazeutische Zusammensetzung nach einem der Ansprüche 27 bis 32, wobei der modifizierte Faktor VII ein humaner ist.

## Revendications

1. Composition pharmaceutique pour inhiber la coagulation du sang in vivo, la composition comprenant un Facteur VII ayant au moine une modification dans son centre catalytique, laquelle modification inhibe sensiblement la capacité dudit Facteur VII modifié à activer les Facteurs X ou IX plasmatiques, en combinaison avec un support physiologiquement acceptable.

2. Composition pharmaceutique selon la revendication 1, dans'laquelle ladite modification comprend la réaction du Facteur VII avec un inhibiteur protéase à sérine.

3. Composition pharmaceutique selon la revendication 2, dans laquelle ledit inhibiteur est un composé organophosphoré, un fluorure de sulfanyle, une peptide-halogénométhylcétone ou un azapeptide.

4. Composition pharmaceutique selon la revendication 2, dans laquelle ledit inhibiteur est une peptide-halogénométhylcétone choisie parmi la D-Phe-Pro-Arg-chlorométhylcétone ou la Danayl-Glu-Gly-Arg-chlorométhylcétone.

5. Composition pharmaceutique selon la revendication 1, dans laquelle la modification du Facteur VII comprend au moins une substitution, une insertion ou une délétion d'acide aminé dans une triade catalytique de Ser, Asp et His.

6. Composition pharmaceutique selon la revendication 5, dans laquelle le Facteur VII est modifié par une substitution au résidu Ser₃₄₄·

7. Composition pharmaceutique selon l'une quelconque des revendications précédentes, dans laquelle le Facteur VII modifié est humain.

8. Facteur VII modifié tel que défini dans l'une quelconque des revendications précédentes, pour une utilisation en thérapie.

9. Utilisation du Facteur VII modifié tel que défini dans l'une quelconque des revendications 1 à 7 dans la fabrication d'un médicament destiné à inhiber la coagulation du sang.

10. Facteur VII modifié qui comprend au moins une substitution, une insertion ou une délétion d'acide aminé dans la triade catalytique de Ser, Asp et His du Facteur VII humain qui inhibe sensiblement la capacité du Facteur VIIa à activer le Facteur X ou IX plasmatique.

11. Facteur VII modifié de la revendication 10, dane lequel la triade catalytique est constituée de Ser₃₄₄, Asp₂₄₂ et His₁₉₃.

12. Facteur VII modifié de la revendication 11, dans lequel la modification d'acide aminé est une substitution.

13. Facteur VII modifié de la revendication 12, dans lequel la substitution est en Ser₃₄₄.

14. Facteur VII modifié de la revendication 13, dans lequel Ser₃₄₄ est substitué par Ala, Gly, Met ou Thr.

15. Facteur VII modifié de la revendication 14, dans lequel Ser₃₄₄ est substitué par Ala.

16. Facteur VII modifié de la revendication 12, dans lequel Asp₂₄₂ est substitué par Glu.

17. Facteur VII modifié de la revendication 12, dans lequel His₁₉₃ est substitué par Lys ou Arg.

18. Facteur VII modifié de l'une quelconque des revendications 10 à 17, clivé en son site d'activation.

19. Facteur VII modifié de l'une quelconque des revendications 10 à 17, qui lie le facteur tissulaire.

20. Facteur VII modifié de l'une quelconque des revendications 10 à 17, qui entre en compétition avec le Facteur VIIa de type sauvage pour la liaison au facteur tissulaire.

21. Facteur VII modifie de l'une quelconque des revendications 10 à 20, pour une utilisation en thérapie.

22. Utilisation du Facteur VII modifié de l'une quelconque des revendications 10 à 20 dans la fabrication d'une composition pharmaceutique destiné à inhiber la coagulation du sang.

23. Molécule polynucléotidique comprenant deux régions codantes de séquences liées de façon opérationnelle codant, respectivement, un pré-pro-peptide et un domaine gla d'une protéine plasmatique dépendante de la vitamine K, et un Facteur VII sans domaine gla ayant au moins une modification d'acide aminé dans une triade catalytique de Ser, Asp et His, où, après expression, ledit polynucléotide code une molécule de Facteur VII modifié qui a une capacité sensiblement réduite à activer les Facteurs X ou IX plasmatiques.

24. Molécule polynucléotidique selon la revendication 23, dans laquelle la triade catalytique ayant la modification d'acide aminé est constituée de Ser₃₄₄, Asp₂₄₂ et His₁₉₃.

25. Lignée cellulaire de mammifère transfectée avec la molécule polynucléotidique de la revendication 23 ou de la revendication 24.

26. Lignée cellulaire selon la revendication 25, dans laquelle la triade catalytique est Ser₃₄₄, Asp₂₄₂ et His₁₉₃ du Facteur VII humain.

27. Composition pharmaceutique comprenant un Facteur VII ayant au moins une modification dans son centre catalytique, laquelle modification inhibe sensiblement la capacité dudit Facteur VII modifié à activer les Facteurs X ou IX plasmatiques, en combinaison avec un support physiologiquement acceptable.

28. Composition pharmaceutique selon la revendication 27, dans laquelle ladite modification comprend la réaction du Facteur VII avec un inhibiteur protéase à sérine.

29. Composition pharmaceutique selon la revendication 28, dans laquelle ledit inhibiteur est un composé organophosphoré, un fluorure de sulfanyle, une peptide-halogénométhylcétone ou un azapeptide.

30. Composition pharmaceutique selon la revendication 28, dans laquelle ledit inhibiteur est une peptide-halogénométhylcétone choisie parmi la D-Phe-Pro-Arg-chlorométhylcétone ou la Dansyl-Glu-Gly-Arg-chlorométhylcétone.

31. Composition pharmaceutique selon la revendication 27, dans laquelle la modification du Facteur VII comprend au moins une substitution, une insertion ou une délétion d'acide aminé dans une triade catalytique de Ser, Asp et His.

32. Composition pharmaceutique selon la revendication 31, dans laquelle le Facteur VII est modifié par une substitution au résidu Ser₃₄₄.

33. Composition pharmaceutique selon l'une quelconque des revendications 27 à 32, dans laquelle le Facteur VII modifié est humain.
